# EUROPÄISCHE PATENTSCHRIFT

(11) **EP 1 180 008 B1**
(45) Veröffentlichungstag und Bekanntmachung des Hinweises auf die Patenterteilung: **07.06.2006**
(21) Anmeldenummer: 01925386.3
(22) Anmeldetag: 07.03.2001
(51) Int. Cl.: A61Q 5/10, A61K 8/49, A61K 8/42

(54) **MITTEL UND VERFAHREN ZUR FÄRBUNG KERATINISCHER FASERN**
AGENT AND METHOD FOR COLOURING KERATIN FIBRES
AGENT ET PROCEDE POUR LA COLORATION DE FIBRES KERATINIQUES

(30) Priorität: 22.03.2000 DE 10014149
(43) Veröffentlichungstag der Anmeldung: 20.02.2002
(73) Patentinhaber: Wella Aktiengesellschaft, 64274 Darmstadt (DE)
(72) Erfinder: FRIESS, Gabriele, 64823 Gross-Umstadt (DE); BAHNMUELLER, Iris, 69469 Weinheim (DE)
(86) Internationale Anmeldenummer: PCT/EP2001/002556
(87) Internationale Veröffentlichungsnummer: WO 2001/070182

(56) Entgegenhaltungen:
- EP-A- 0 692 245
- EP-A- 0 891 971
- DE-U- 29 909 427
- US-A- 5 961 667

## Beschreibung

Die vorliegende Erfindung betrifft ein Mittel zur Färbung von keratinischen Fasern, insbesonders von menschlichen Haaren, welches mindestens ein 4,5-Diaminopyrazol-Derivat und mindestens ein Phenylhamstoff-Derivat enthält. Die Färbungen der keratinischen Fasern werden ausgeführt, indem die oben erwähnte Zusammensetzung mit einem Oxidationsmittel vermischt wird, auf die Faser aufgetragen wird und nach einer bestimmten Einwirkungszeit wieder ausgewaschen wird.

Die Verwendung von Phenylhamstoffen in Färbemitteln ist aus der Literatur bekannt. So werden in der DE-PS 18 10 191 Färbemittel beschrieben, welche eine Kombination von Phenylharnstoffen mit 1,4-Diaminobenzol oder 2,5-Diaminotoluol-Sulfat enthalten. Diese Mittel ermöglichen jedoch nur blaue, grüne oder stark blaustichige violette Färbungen. Aus der DE-GM 299 094 27 sind Färbemittel bekannt, welche unter anderem eine Kombination aus bestimmten Pyrazolen und N-(3-Dimethylamino)-phenylharnstoff enthalten. Diese Mittel ergeben jedoch ebenfalls keine reinen Rottöne.

Es bestand daher weiterhin ein Bedarf nach Färbemitteln auf der Basis von Phenylhamstoff-Derivaten, welche leuchtende, intensive, reine Rottöne ermöglichen.

Es wurde nunmehr überraschend gefunden, dass bei Verwendung einer Kombination aus 4,5-Diaminopyrazolen der Formel (I) und Phenylhamstoffen der Formel (II) eine intensiv leuchtend rote oxidative Haarfärbung möglich ist, wobei auch ohne den Zusatz weiterer Oxidationsfarbstoffvorstufen intensive, reine Rottöne erhältlich sind.

Gegenstand der vorliegenden Anmeldung ist daher ein Mittel zum Färben von Keratinfasem, welches vor der Anwendung mit einem Oxidationsmittel vermischt wird und dadurch gekennzeichnet ist, dass es in einem geeigneten kosmetischen Träger eine Kombination aus
- mindestens einem 4,5-Diaminopyrazol-Derivat der allgemeinen Formel (I) oder dessen physiologisch verträglichen Salzen, 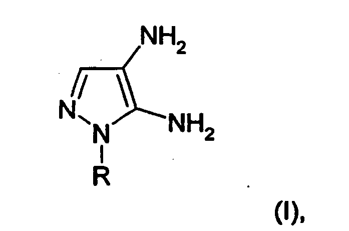 worin R eine geradkettige oder verzweigte Alkylgruppe mit 1 bis 6 Kohlenstoffatomen; eine geradkettige oder verzweigte Monohydroxyalkylgruppe mit 1 bis 6 Kohlenstoffatomen; eine geradkettige oder verzweigte Polyhydroxyalkylgruppe mit 2 bis 6 Kohlenstoffatomen; eine geradkettige oder verzweigte Monoaminoalkylgruppe mit 1 bis 6 Kohlenstoffatomen; eine geradkettige oder verzweigte Polyaminoalkylgruppe mit 2 bis 6 Kohlenstoffatomen; eine mit einem Halogenatom, einer geradkettigen oder verzweigten Alkylgruppe mit 1 bis 6 Kohlenstoffatomen oder einer geradkettigen oder verzweigten Alkoxygruppe mit 1 bis 6 Kohlenstoffatomen substituierte Benzylgruppe oder eine unsubstituierte Benzylgruppe darstellt; und
- mindestens einem Phenylhamstoff-Derivat der allgemeinen Formel (II) oder dessen physiologisch verträglichen Salzen, 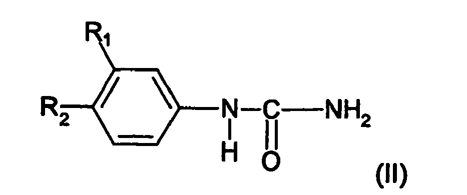
worin **R1** eine Hydroxylgruppe oder eine geradkettige oder verzweigte Alkoxygruppe mit 1 bis 6 Kohlenstoffatomen bedeutet; und **R2** ein Wasserstoffatom, ein Halogenatom, eine geradkettige oder verzweigte Alkylgruppe mit 1 bis 6 Kohlenstoffatomen, eine geradkettige oder verzweigte Alkoxygruppe mit 1 bis 6 Kohlenstoffatomen, eine geradkettige oder verzweigte Hydroxyalkylgruppe mit 1 bis 6 Kohlenstoffatomen oder eine geradkettige oder verzweigte Polyhydroxyalkylgruppe mit 2 bis 6 Kohlenstoffatomen bedeutet; enthält.

Besonders bevorzugte Derivate der allgemeinen Formel (I) sind die folgenden Verbindungen oder deren physiologisch verträglichen Salze: 4,5-Diamino-1-methyl-1 H-pyrazol; 4,5-Diamino-1-(4'-methylbenzyl)-pyrazol; 4,5-Diamino-1-(2'-hydroxyethyl)-1H-pyrazol; 4,5-Diamino-1-benzyl-1 H-pyrazol; 4,5-Diamino-1-ethyl-1 H-pyrazol; 4,5-Diamino-1-isopropyl-1H-pyrazol; 4,5-Diamino-1-(4'-methoxybenzyl)-1H-pyrazol; 4,5-Diamino-1 H-pyrazol; 4,5-Diamino-l-(3'-methoxybenzyl)-1 H-pyrazol und 4,5-Diamino-1-(4'-chlorbenzyl)-1H-pyrazol.

Besonders bevorzugte Derivate der allgemeinen Formel (II) sind die folgenden Verbindungen oder deren physiologisch verträglichen Salze: 3-Ureido-phenol, 2-Chlor-5-ureido-phenol, 2-Methyl-5-ureido-phenol und 2-Methoxy-5-ureido-phenol.

Obwohl die erfindungsgemässe Farbstoftkombination bereits ohne Zusatz weiterer Farbstoffe zu ausgezeichneten Rotfärbungen führt, können dem erfindungsgemässen Färbemittel zur Abrundung des Farbergebnisses sowie zur Erzeugung von speziellen Farbeffekten weitere Oxidationsfarbstoffvorstufen, wie zum Beispiel Derivate des p-Phenylendiamins wie 2-(2',5'-Diaminophenyl)ethanol, Resorcinderivate wie Resorcin, 2-Methylresorcin oder 4-Chlorresorcin, Amino- und Hydroxyderivate des 1,3-Benzodioxols, Naphthalinderivate wie 1-Hydroxynaphthalin, 1,5- Di-hydroxynaphthalin oder 1,7-Dihydroxynaphthalin, sowie direktziehende Farbstoffe, wie zum Beispiel 2-Amino-4,6-dinitrophenol, 2-Ethylamino-4,6-dinitrophenol, 2-[(2'-Hydroxyethyl)-amino]-4,6-dinitrophenol, 2-Amino-6-chlor-4-nitrophenol oder 2-Chlor-6-ethylamino-4-nitrophenol, zugesetzt werden.

Die Verbindungen der Formel (I) und (II) können ebenso wie die vorstehend beschriebenen oxidativen und direktziehenden Farbstoffe sowohl in Form der freien Base als auch in Form ihrer physiologisch verträglichen Salzen mit anorganischen oder organischen Säuren, wie zum Beispiel Salzsäure, Phosphorsäure, Schwefelsäure, Essigsäure, Milchsäure oder Zitronensäure, insbesondere in Form der Hydrochloride oder Sulfate, eingesetzt werden. Ebenfalls können diese Verbindungen im Fall von Phenolen als Alkaliphenolate vorliegen.

Die Gesamtkonzentration an Farbstoffvorstufen beträgt in dem erfindungsgemässen Färbemittel etwa 0,1 bis 20 Gewichtsprozent, vorzugsweise 0,2 bis 6 Gewichtsprozent. Die Konzentration der Verbindungen der Formel (I) und (II) sowie der Oxidationsfarbstoffvorstufen und direktziehenden Farbstoffe beträgt jeweils etwa 0,01 bis 5 Gewichtsprozent, vorzugsweise 0,1 bis 4 Gewichtsprozent.

Darüberhinaus können in dem erfindungsgemässen Färbemittel als Bestandteil des kosmetischen Trägers übliche kosmetische Zusätze, beispielsweise Antioxidantien wie Ascorbinsäure, Thioglykolsäure oder Natriumsulfit; Parfümöle; Komplexbildner, Netzmittel; Emulgatoren; Verdicker; Pflegestoffe und andere für kosmetische Mittel geeignete Hilfs- und Zusatzstoffe enthalten sein.

Die Zubereitungsform für das erfindungsgemässe Färbemittel (und zwar sowohl vor als auch nach dem Vermischen mit dem Oxidationsmittel) kann beispielsweise eine Lösung, insbesondere eine wässrige oder wässrig-alkoholische Lösung; eine Creme; ein Gel oder eine Emulsion sein. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, n-Propanol und Isopropanol oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, femer Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (bezogen auf die Farbträgermasse), die Verdicker in einer Menge von etwa 0,1 bis 25 Gewichtsprozent (bezogen auf die Farbträgermasse) und die Pflegestoffe in einer Konzentration von etwa 0,1 bis 5,0 Gewichtsprozent (bezogen auf die Farbträgermasse).

Unmittelbar vor der Anwendung wird das erfindungsgemässe Haarfärbemittel mit einem flüssigen Oxidationsmittel in einem geeigneten Verhältnis vermischt. Das Färbemittel und das Oxidationsmittel werden hierbei vorzugsweise in einem Gewichtsverhältnis von etwa 5:1 bis 1:3 miteinander vermischt, wobei ein Gewichtsverhältnis von 1:1 bis 1:2 besonders bevorzugt ist.

Der pH-Wert des gebrauchsfertigen erfindungsgemässen Haarfärbemittel stellt sich bei der Mischung der vorzugsweise alkalisch eingestellten Farbträgermasse mit dem meist sauer eingestellten Oxidationsmittel auf einen pH-Wert ein, der durch die Alkalimengen in der Farbträgermasse und die Säuremengen im Oxidationsmittel sowie durch das Mischungsverhältnis beinflußt wird. Der pH-Wert des fertigen Haarfärbemittels liegt bei etwa 3 bis 11, vorzugsweise 5 bis 9.

Für die Einstellung des jeweiligen pH-Wertes der Farbträgermasse und des Oxidationsmittels können je nach dem gewünschten pH-Wert organische und anorganische Säuren, wie zum Beispiel Phosphorsäure, Ascorbinsäure und Milchsäure, oder Alkalien wie zum Beispiel Monoethanolamin, Triethanoiamin, 2-Amino-2-methyl-1-propanol, Ammoniak, Natronlauge, Kalilauge oder Tris-(hydroxymethyl)-aminomethan, verwendet werden.

Für die Anwendung zur oxidativen Färbung von Haaren vermischt man das vorstehend beschriebene erfindungsgemässe Färbemittel unmittelbar vor dem Gebrauch mit dem Oxidationsmittel und trägt eine für die Haarfärbebehandlung ausreichende Menge, je nach Haarfülle im allgemeinen etwa 60 bis 200 Gramm des erhaltenen gebrauchsfertigen Oxidationsfärbemittels auf das Haar auf.

Als Oxidationsmittel kommen hauptsächlich Wasserstoffperoxid oder dessen Additionsverbindungen an Hamstoff, Melamin oder Natriumbromat in Form einer 1 bis 12prozentigen, vorzugsweise 6prozentigen, wässrigen Lösung in Betracht, wobei Wasserstoffperoxid besonders bevorzugt wird.

Man lässt das erfindungsgemäße Färbemittel bei 15 bis 50 Grad Celsius etwa 10 bis 45 Minuten lang, vorzugsweise 25 bis 40 Minuten lang (insbesondere 30 Minuten lang), auf das Haar einwirken, spült sodann das Haar mit Wasser und trocknet es. Gegebenenfalls wird im Anschluss an diese Spülung mit einem Shampoo gewaschen und eventuell mit einer schwachen organischen Säure, wie zum Beispiel Zitronensäure oder Weinsäure, nachgespült. Anschliessend wird das Haar getrocknet.

Die nachfolgenden Beispiele sollen den Gegenstand näher erläutern, ohne ihn auf diese Beispiele zu beschränken.

### Beispiele

| **Beispiel 1:** | Oxidationshaarfärbemittel, alkalisch |
|---|---|
| 10,00 g | Isopropanol |
| 10,00 g | Laurylalkohol-diglykolether-sulfat-Natriumsalz, (28prozentige wässrige Lösung) |
| 10,00 g | Ammoniak (25prozentige wässrige Lösung) |
| 0,30 g | Ascorbinsäure |
| 1,50 g | 4,5-Diamino-1-(4'-methylbenzyl) H-pyrazol-sulfat |
| 0,92 g | 3-Ureido-Phenol |
| ad 100,00 g | Wasser, vollentsalzt |

Der pH-Wert der Haafärbelösung beträgt 10,5.

Vor der Anwendung werden 10 g der oben beschriebenen Haarfärbelösung mit 10 g einer Wasserstoffperoxid-Lösung (6prozentige Lösung in Wasser) vermischt. Das so erhaltene gebrauchsfertige Oxidationsfärbemittel mit einem pH-Wert von 9,5-9,8 wird auf die Haare aufgetragen. Nach einer Einwirkungszeit von 30 Minuten bei 40 °C wird das Haar mit Wasser ausgespült, schamponiert und getrocknet. Das Haar erhält eine intensiv himbeerrote Färbung.

| **Beispiel 2:** | Oxidationshaarfärbemittel, alkalisch |
|---|---|
| 2,00 g | 4,5-Diamino-1 -(2'-hydroxyethyl)-1 H-pyrazol-sulfat |
| 1,56 g | 2-Chlor-5-ureido-phenol |
| 15,00 g | Cetylalkohol |
| 3,50 g | Laurylalkohol-diglykolether-sultat-Natriumsalz, (28prozentige wässrige Lösung) |
| 3,00 g | Ammoniak (25prozentige wässrige Lösung) |
| 0,30 g | Natriumsulfit, wasserfrei |
| ad 100,00 g | Wasser, vollentsalzt |

Der pH-Wert der Haafärbecreme beträgt 10,5.

Unmittelbar vor der Anwendung werden 10 g der oben beschriebenen Haarfärbecreme mit 10 g einer Wasserstoffperoxid-Lösung (6prozentige Lösung in Wasser) vermischt. Das so erhaltene gebrauchsfertige Oxidationsfärbemittel mit einem pH-Wert von 9,5-9,8 wird auf die Haare aufgetragen und nach einer Einwirkungszeit von 30 Minuten bei 40 °C mit Wasser ausgespült und getrocknet. Das so behandelte Haar hat eine leuchtend pink-farbene Färbung erhalten.

| **Beispiel 3:** | Oxidationshaarfärbemittel, alkalisch |
|---|---|
| 2,05 g | 4,5-Diamino-1-benzyl-1 H-pyrazol-sulfat |
| 1,53 g | 2-Methyl-5-ureido-phenol |
| 0,40 g | Natriumhydroxid, fest |
| 0,60 g | Ascorbinsäure |
| 7,00 g | Isopropanol |
| 15,00 g | Ölsäure |
| 10,00 g | Ammoniak (25prozentige wässrige Lösung) |
| ad 100,00 g | Wasser, vollentsalzt |

Der pH-Wert des Haafärbegels beträgt 10,5.

Unmittelbar vor der Anwendung werden 10 g der oben beschriebenen Haarfärbegels mit 10 g einer Wasserstoffperoxid-Lösung (6prozentige Lösung in Wasser) vermischt. Das so erhaltene gebrauchsfertige Oxidationsfärbemittel mit einem pH-Wert von 9,5-9,8 wird auf die Haare aufgetragen. Nach einer Einwirkungszeit von 40 Minuten bei 35 Grad Celsius wird mit Wasser gespült und sodann getrocknet. Man erhält eine leuchtend rotorange Haarfärbung.

| **Beispiel 4:** | Oxidationshaarfärbemittel, alkalisch |
|---|---|
| 10,00 g | Isopropanol |
| 10,00 g | Laurylalkohol-diglykolether-sulfat-Natriumsalz, (28prozentige wässrige Lösung) |
| 10,00 g | Ammoniak (25prozentige wässrige Lösung) |
| 0,30 g | Ascorbinsäure |
| 3,00 g | 4,5-Diamino-1-isopropyl-1 H-pyrazol-sulfat |
| 1,92 g | 3-Ureido-phenol |
| ad 100,00 g | Wasser, vollentsalzt |

Der pH-Wert der Haafärbelösung beträgt 10,5.

Unmittelbar vor der Anwendung werden 10 g der oben beschriebenen Haarfärbelösung mit 10 g einer Wasserstoffperoxid-Lösung (6prozentige Lösung in Wasser) vermischt. Das so erhaltene gebrauchsfertige Oxidationsfärbemittel mit einem pH-Wert von 9,5-9,8 wird auf die Haare aufgetragen. Nach einer Einwirkungszeit von 30 Minuten bei 40 Grad Celsius wird mit Wasser gespült und sodann getrocknet. Das Haar erhält eine intensiv himbeerrote Färbung.

| **Beispiel 5:** | Oxidationshaarfärbemittel, sauer |
|---|---|
| 10,00 g | Isopropanol |
| 10,00 g | Laurylalkohol-diglykolether-sulfat-Natriumsalz, (28prozentige wässrige Lösung) |
| 10,00 g | Ammoniak (25prozentige wässrige Lösung) |
| 0,30 g | Ascorbinsäure |
| 1,50 g | 4,5-Diamino-1-(4'-methylbenzyl)-1H-pyrazol-sulfat |
| 0,92 g | 3-Ureido-Phenol |
| ad 100,00 g | Wasser, vollentsalzt |

Der pH-Wert der Haafärbelösung beträgt 10,5.

Vor der Anwendung werden 10 g der oben beschriebenen Haarfärbelösung mit 10 g einer Wasserstoffperoxid-Lösung (6prozentige Lösung in Wasser) vermischt und das so erhaltene gebrauchsfertige Oxidationsfärbemittel mit Phosphorsäure auf einen pH-Wert von 6,8 eingestellt. Anschließend wird das Oxidationsfärbemittel auf die Haare aufgetragen. Nach einer Einwirkungszeit von 30 Minuten bei 40°C wird das Haar mit Wasser ausgespült, schamponiert und getrocknet. Das Haar erhält eine intensiv himbeerrote Färbung.

| **Beispiel 6:** | Oxidationshaarfärbemittel, sauer |
|---|---|
| 2,00 g | 4,5-Diamino-1-(2'-hydroxyethyl)-1 H-pyrazol-sulfat |
| 1,56 g | 2-Chlor-5-ureido-phenol |
| 15,00 g | Cetylalkohol |
| 3,50 g | Laurylalkohol-diglykolether-sulfat-Natriumsalz, (28prozentige wässrige Lösung) |
| 3,00 g | Ammoniak (25prozentige wässrige Lösung) |
| 0,30 g | Natriumsulfit, wasserfrei |
| ad 100,00 g | Wasser, vollentsalzt |

Der pH-Wert der Haafärbecreme beträgt 10,5.

Unmittelbar vor der Anwendung werden 10 g der oben beschriebenen Haarfärbecreme mit 10 g einer Wasserstoffperoxid-Lösung (6prozentige Lösung in Wasser) vermischt und das so erhaltene gebrauchsfertige Oxidationsfärbemittel mit Phosphorsäure auf einen pH-Wert von 6,8 eingestellt. Anschließend wird das Oxidationsfärbemittel auf die Haare aufgetragen und nach einer Einwirkungszeit von 30 Minuten bei 40 °C mit Wasser ausgespült und getrocknet. Das so behandelte Haar hat eine leuchtend pink-farbene Färbung erhalten.

## Patentansprüche

1. Mittel zum Färben von Keratinfasem, welches vor der Anwendung mit einem Oxidationsmittel vermischt wird und **dadurch gekennzeichnet ist, dass** es in einem geeigneten kosmetischen Träger eine Kombination aus
- mindestens einem 4,5-Diaminopyrazol-Derivat der allgemeinen Formel (I) oder dessen physiologisch verträglichen Salzen, 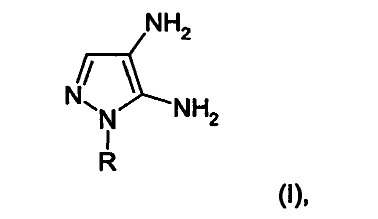 worin R eine geradkettige oder verzweigte Alkylgruppe mit 1 bis 6 Kohlenstoffatomen; eine geradkettige oder verzweigte Monohydroxyalkylgruppe mit 1 bis 6 Kohlenstoffatomen; eine geradkettige oder verzweigte Polyhydroxyalkylgruppe mit 2 bis 6 Kohlenstoffatomen; eine geradkettige oder verzweigte Monoaminoalkylgruppe mit 1 bis 6 Kohlenstoffatomen; eine geradkettige oder verzweigte Polyaminoalkylgruppe mit 2 bis 6 Kohlenstoffatomen; eine mit einem Halogenatom, einer geradkettigen oder verzweigten Alkylgruppe mit 1 bis 6 Kohlenstoffatomen oder einer geradkettigen oder verzweigten Alkoxygruppe mit 1 bis 6 Kohlenstoffatomen substituierte Benzylgruppe oder eine unsubstituierte Benzylgruppe darstellt; und
- mindestens einem Phenylhamstoff-Derivat der allgemeinen Formel (II) oder dessen physiologisch verträglichen Salzen, 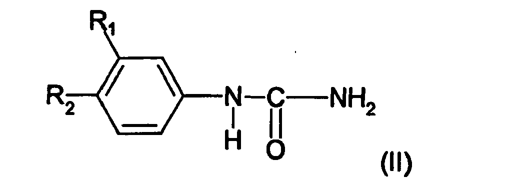
worin **R1** eine Hydroxylgruppe oder eine geradkettige oder verzweigte Alkoxygruppe mit 1 bis 6 Kohlenstoffatomen bedeutet; und
**R2** ein Wasserstoffatom, ein Halogenatom, eine geradkettige oder verzweigte Alkylgruppe mit 1 bis 6 Kohlenstoffatomen, eine geradkettige oder verzweigte Alkoxygruppe mit 1 bis 6 Kohlenstoffatomen, eine geradkettige oder verzweigte Hydroxyalkylgruppe mit 1 bis 6 Kohlenstoffatomen oder eine geradkettige oder verzweigte Polyhydroxyalkylgruppe mit 2 bis 6 Kohlenstoffatomen bedeutet; enthält.

2. Mittel gemäß Anspruch 1, **dadurch gekennzeichnet, dass** das 4,5-Diaminopyrazol-Derivat der Formel (I) ausgewählt ist aus 4,5-Diamino-1-methyl-1 H-pyrazol; 4,5-Diamino-1 -(4'-methylbenzyl)- pyrazol; 4,5-Diamino-1-(2'-hydroxyethyl)-1H-pyrazol; 4,5-Diamino-1-benzyl-1 H-pyrazol; 4,5-Diamino-1-ethyl-1H-pyrazol; 4,5-Diamino-1-isopropyl-1H-pyrazol; 4,5-Diamino-1-(4'-methoxybenzyl)-1 H-pyrazol; 4,5-Diamino-1 H-pyrazol; 4,5-Diamino-1-(3'-methoxybenzyl)-1H-pyrazol und 4,5-Diamino-1-(4'-chlorbenzyl)-1 H-pyrazol oder deren physiologisch verträglichen Salzen.

3. Mittel gemäß Anspruch 1 oder 2, **dadurch gekennzeichnet, dass** das Phenylhamstoff-Derivat der Formel (II) ausgewählt ist aus 3-Ureidophenol, 2-Chlor-5-ureido-phenol, 2-Methyl-5-ureido-phenol und 2-Methoxy-5-ureido-phenol oder deren physiologisch verträglichen Salzen.

4. Mittel gemäß einem der Ansprüche 1 bis 3, **dadurch gekennzeichnet, dass** es zusätzlich weitere Oxidationsfarbvorstufen aus der Gruppe bestehend aus p-Phenylendiaminderivaten, Resorcinderivaten, Amino- und Hydroxyderivaten des 1,3-Benzodioxols und Naphthalinderivaten enthält.

5. Mittel gemäß einem der Ansprüche 1 bis 4, **dadurch gekennzeichnet, dass** es zusätzlich direktziehende Farbstoffe enthält.

6. Mittel gemäß einem der Ansprüche 1 bis 5, **dadurch gekennzeichnet, dass** die Verbindungen der Formel (1) und (II) jeweils in einer Menge von 0,01 bis 5 Gewichtsprozent enthalten sind.

7. Mittel gemäß einem der Ansprüche 1 bis 6, **dadurch gekennzeichnet, dass** die Gesamtkonzentration der Farbstoffe 0,1 bis 20 Gewichtsprozent beträgt.

8. Mittel gemäß einem der Ansprüche 1 bis 7, **dadurch gekennzeichnet, dass** es mit dem Oxidationsmittel in einem Verhältnis von 5:1 bis 1: 3 vermischt wird.

9. Mittel gemäß einem der Ansprüche 1 bis 8, **dadurch gekennzeichnet, dass** es einen pH-Wert von 3 bis 11 aufweist.

10. Verfahren zum Färben von Haaren, **dadurch gekennzeichnet, dass** ein Haarfärbemittel nach einem der Ansprüche 1 bis 9 auf das Haar aufgetragen wird, bei einer Temperatur von 15 bis 50°C 10 bis 45 Minuten lang einwirken gelassen wird, das Haar anschliessend mit Wasser gespült wird, gegebenenfalls schamponiert wird und sodann getrocknet wird.

## Claims

1. Agent for colouring keratin fibres, which is mixed prior to use with an oxidizing agent and is **characterized in that** it comprises, in a suitable cosmetic carrier, a combination of
- at least one 4,5-diaminopyrazole derivative of the general formula (I) or physiologically compatible salts thereof, 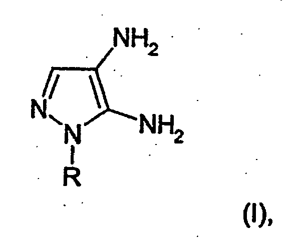 in which R is a straight-chain or branched alkyl group having 1 to 6 carbon atoms; a straight-chain or branched monohydroxyalkyl group having 1 to 6 carbon atoms; a straight-chain or branched polyhydroxyalkyl group having 2 to 6 carbon atoms; a straight-chain or branched monoaminoalkyl group having 1 to 6 carbon atoms; a straight-chain or branched polyaminoalkyl group having 2 to 6 carbon atoms; a benzyl group substituted by a halogen atom, a straight-chain or branched alkyl group having 1 to 6 carbon atoms or a straight-chain or branched alkoxy group having 1 to 6 carbon atoms, or an unsubstituted benzyl group; and
- at least one phenylurea derivative of the general formula (II) or physiologically compatible salts thereof, 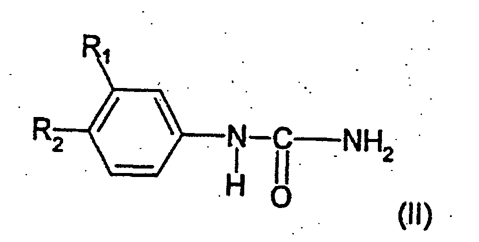
in which **R1** is a hydroxyl group or a straight-chain or branched alkoxy group having 1 to 6 carbon atoms; and **R2** is a hydrogen atom, a halogen atom, a straight-chain or branched alkyl group having 1 to 6 carbon atoms, a straight-chain or branched alkoxy group having 1 to 6 carbon atoms, a straight-chain or branched hydroxyalkyl group having 1 to 6 carbon atoms or a straight-chain or branched polyhydroxyalkyl group having 2 to 6 carbon atoms.

2. Agent according to Claim 1, **characterized in that** the 4,5-diaminopyrazole derivative of the formula (I) is chosen from 4,5-diamino-1-methyl-1H-pyrazole; 4,5-diamino-1-(4'-methylbenzyl)pyrazole; 4,5-diamino-1-(2'-hydroxyethyl)-1H-pyrazole; 4,5-diamino-1-benzyl-1H-pyrazole; 4,5-diamino-1-ethyl-1H-pyrazole; 4,5-diamino-1-isopropyl-1H-pyrazole; 4,5-diamino-1-(4'-methoxybenzyl)-1H-pyrazole; 4,5-diamino-1H-pyrazole; 4,5-diamino-1-(3'-methoxybenzyl)-1H-pyrazole and 4,5-diamino-1-(4'-chlorobenzyl)-1H-pyrazole or physiologically compatible salts thereof.

3. Agent according to Claim 1 or 2, **characterized in that** the phenylurea derivative of the formula (II) is chosen from 3-ureidophenol, 2-chloro-5-ureidophenol, 2-methyl-5-ureidophenol and 2-methoxy-5-ureidophenol or physiologically compatible salts thereof.

4. Agent according to one of Claims 1 to 3, **characterized in that** it additionally comprises further oxidation dye precursors from the group consisting of p-phenylenediamine derivatives, resorcinol derivatives, amino and hydroxy derivatives of 1,3-benzodioxol and naphthalene derivatives.

5. Agent according to one of Claims 1 to 4, **characterized in that** it additionally comprises direct dyes.

6. Agent according to one of Claims 1 to 5, **characterized in that** the compounds of the formula (I) and (II) are each present in an amount of from 0.01 to 5 per cent by weight.

7. Agent according to one of Claims 1 to 6, **characterized in that** the total concentration of the dyes is 0.1 to 20 per cent by weight.

8. Agent according to one of Claims 1 to 7, **characterized in that** it is mixed with the oxidizing agent in a ratio of from 5:1 to 1:3.

9. Agent according to one of Claims 1 to 8, **characterized in that** it has a pH of from 3 to 11.

10. Method for colouring hair, **characterized in that** a hair colorant according to one of Claims 1 to 9 is applied to the hair and left to act at a temperature of from 15 to 50°C for 10 to 45 minutes, and the hair is then rinsed with water, optionally shampooed and then dried.

## Revendications

1. Agent pour la teinture de fibres kératiniques qui est mélangé avant l'utilisation avec un oxydant et qui est **caractérisé en ce qu'**il contient, dans un support cosmétique approprié, une combinaison
- d'au moins un dérivé de 4,5-diaminopyrazole de formule générale (I) ou ses sels physiologiquement acceptables, 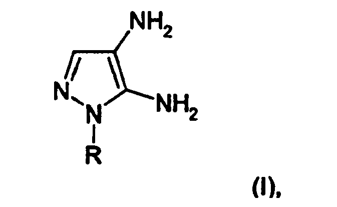 dans laquelle R représente un groupe alkyle linéaire ou ramifié comprenant 1 à 6 atomes de carbone ; un groupe monohydroxyalkyle linéaire ou ramifié comprenant 1 à 6 atomes de carbone ; un groupe polyhydroxyalkyle linéaire ou ramifié comprenant 2 à 6 atomes de carbone ; un groupe monoaminoalkyle linéaire ou ramifié comprenant 1 à 6 atomes de carbone ; un groupe polyaminoalkyle linéaire ou ramifié comprenant 2 à 6 atomes de carbone ; un groupe benzyle substitué par un atome d'halogène, un groupe alkyle linéaire ou ramifié comprenant 1 à 6 atomes de carbone ou un groupe alcoxy linéaire ou ramifié comprenant 1 à 6 atomes de carbone ou un groupe benzyle non substitué ; et
- d'au moins un dérivé de phénylurée de formule générale (II) ou ses sels physiologiquement acceptables, 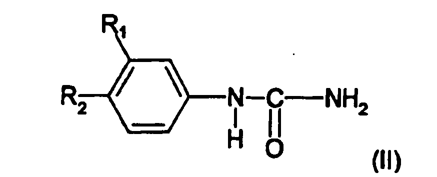
dans laquelle R1 signifie un groupe hydroxyle ou un groupe alcoxy linéaire ou ramifié comprenant 1 à 6 atomes de carbone ; et R2 signifie un atome d'hydrogène, un atome d'halogène, un groupe alkyle linéaire ou ramifié comprenant 1 à 6 atomes de carbone, un groupe alcoxy linéaire ou ramifié comprenant 1 à 6 atomes de carbone, un groupe hydroxyalkyle linéaire ou ramifié comprenant 1 à 6 atomes de carbone ou un groupe polyhydroxyalkyle linéaire ou ramifié comprenant 2 à 6 atomes de carbone.

2. Agent selon la revendication 1, **caractérisé en ce que** le dérivé de 4,5-diaminopyrazole de formule (I) est choisi parmi le 4,5-diamino-1-méthyl-1H-pyrazole ; le 4,5-diamino-1-(4'-méthylbenzyl)-pyrazole ; le 4,5-diamino-1-(2'-hydroxyéthyl)-1H-pyrazole ; le 4,5-diamino-1-benzyl-1H-pyrazole ; le 4,5-diamino-1-éthyl-1H-pyrazole ; le 4,5-diamino-1-isopropyl-1H-pyrazole ; le 4,5-diamino-1-(4'-méthoxybenzyl)-1H-pyrazole ; le 4,5-diamino-1H-pyrazole ; le 4,5-diamino-1-(3'-méthoxybenzyl)-1H-pyrazole et le 4,5-diamino-1-(4'-chlorobenzyl)-1H-pyrazole ou leurs sels physiologiquement acceptables.

3. Agent selon la revendication 1 ou 2, **caractérisé en ce que** le dérivé de phénylurée de formule (II) est choisi parmi le 3-uréidophénol, le 2-chloro-5-uréidophénol, le 2-méthyl-5-uréido-phénol et le 2-méthoxy-5-uréido-phénol ou leurs sels physiologiquement acceptables.

4. Agent selon l'une quelconque des revendications 1 à 3, **caractérisé en ce qu'**il contient en outre d'autres précurseurs de colorants d'oxydation du groupe constitué par les dérivés de p-phénylènediamine, les dérivés de résorcinol, les dérivés amino et hydroxy du 1,3-benzodioxol et les dérivés de naphtalène.

5. Agent selon l'une quelconque des revendications 1 à 4, **caractérisé en ce qu'**il contient en outre des colorants montant directement sur la fibre.

6. Agent selon l'une quelconque des revendications 1 à 5, **caractérisé en ce que** les composés de formule (I) et (II) sont à chaque fois contenus en une quantité de 0,01 à 5% en poids.

7. Agent selon l'une quelconque des revendications 1 à 6, **caractérisé en ce que** la concentration totale en colorant est de 0,1 à 20% en poids.

8. Agent selon l'une quelconque des revendications 1 à 7, **caractérisé en ce qu'**il est mélangé avec un oxydant dans un rapport de 5:1 à 1:3.

9. Agent selon l'une quelconque des revendications 1 à 8, **caractérisé en ce qu'**il présente un pH de 3 à 11.

10. Procédé pour la teinture de cheveux, **caractérisé en ce qu'**on applique un agent de teinture pour cheveux selon l'une quelconque des revendications 1 à 9 sur les cheveux, on le laisse agir à une température de 15 à 50°C pendant 10 à 45 minutes, on rince ensuite les cheveux avec de l'eau, on les shampouine le cas échéant puis on les sèche.
